# EUROPÄISCHE PATENTSCHRIFT

(11) **EP 0 690 058 B1**
(45) Veröffentlichungstag und Bekanntmachung des Hinweises auf die Patenterteilung: **08.09.1999**
(21) Anmeldenummer: 95810413.5
(22) Anmeldetag: 20.06.1995
(51) Int. Cl.: C07D 487/04, C09B 57/00, C08K 5/3415

(54) **Neue Kristallmodifikation eines Diketopyrrolopyrrolpigments**
Crystal modification of a diketopyrrolopyrrole pigment
Modification cristalline d'un pigment de dicétopyrrolopyrrole

(30) Priorität: 29.06.1994 CH 207594
(43) Veröffentlichungstag der Anmeldung: 03.01.1996
(73) Patentinhaber: Ciba Specialty Chemicals Holding Inc., 4057 Basel (CH)
(72) Erfinder: Hao, Zhimin, Dr., CH-1723 Marly (CH); Schlöder, Ingo, CH-1753 Matran (CH); Iqbal, Abul, Dr., CH-1732 Arconciel (DE)

(56) Entgegenhaltungen:
- EP-A- 0 094 911
- ACTA CRYSTALLOGRAPHICA, Bd. B48, Nr. 5, Oktober 1992 Seiten 696-700, J. MIZUGUCHI ET AL.
- ANGEWANDTE CHEMIE, Bd. 101, Nr. 4, 1989 Seiten 497-499, H. LANGHALS ET AL.

## Beschreibung

Die vorliegende Anmeldung betrifft eine neue Kristallmodifikation von 1,4-Diketo-3,6-bis(4-chlorophenyl)-pyrrolo[3,4-c]pyrrol, ihre Herstellung sowie die Verwendung dieses neuen Produkts als Pigment.

Es ist allgemein bekannt, dass eine Reihe von Vertretern verschiedener organischer Pigmentklassen polymorph sind. Trotz chemisch gleicher Zusammensetzung treten solche Pigmente in zwei oder mehr Kristallmodifikationen auf. Dies ist insbesondere bei Phthalocyanin-, Chinacridon- und einigen Azopigmenten der Fall (vgl. z.B. W. Herbst, K. Hunger, Industrielle Organische Pigmente (1987), 43-45, 431-433, 458-459). Von einigen anderen Pigmenten ist hingegen nur eine einzige Kristallmodifikation bekannt. Trotz mehrerer Versuche ist es bisher z.B. nicht gelungen von irgendeinem der seit einigen Jahren bekannten und beispielsweise in den US-Patenten 4 415 685 und 4 579 949 beschriebenen Diketopyrrolopyrrolpigmente eine zweite Kristallmodifikation zu erhalten.

Vor kurzem wurde gefunden, dass Abgangsgruppen, wie z.B. solche der Formel 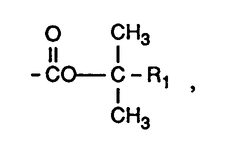 worin R₁ C₁-C₆-Alkyl ist, auch in unlösliche Stoffe, wie die Diketopyrrolopyrrolpigmente, unter Bildung von löslichen Carbamaten der Grundstruktur 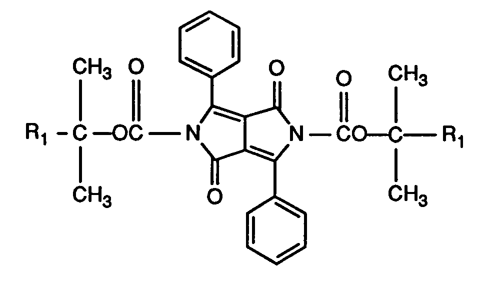 leicht eingeführt werden können und, dass durch thermische (Aufheizen auf Temperaturen zwischen 50 und 400°C), chemische (mit organischen oder anorganischen Säuren oder Basen) oder photolytische (Belichtung z.B. mit Wellenlängen unter 375 nm) Behandlung das ursprüngliche Pigment wieder zurückgebildet werden kann. Diese Untersuchungen werden in Parallelpatentanmeldungen (Anmeldedatum Oktober 1993) beschrieben.

Ganz überraschend ist nun gefunden worden, dass im Falle des Diketopyrrolopyrrols der Formel 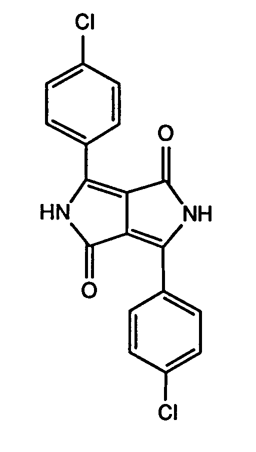 die obenerwähnte Rückbildung zum (N-unsubstituierten) Pigment durch chemische Behandlung unter bestimmten Bedingungen nicht zur bisher bekannten, sondern zu einer neuen Kristallmodifikation führt. Die neue Modifikation, im folgenden β-Modifikation genannt, unterscheidet sich von der bekannten Modifikation, im folgenden α-Modifikation genannt, durch ein bestimmtes, verschiedenes Röntgenbeugungsdiagramm, aber auch durch eine für die Verwendung als Pigment interessante Nuancenverschiebung nach gelbrot.

Die vollständigen Röntgenbeugungsdiagramme werden nach üblichen Methoden mit Hilfe eines Siemens D500® Röntgen-Diffraktometers (CuK_{α}-Strahlung) bestimmt.

Das Röntgenbeugungsdiagramm der weniger gelblichen bekannten α-Modifikation ist durch folgende Beugungslinien

| Netzebenenabstände (d-Werte in Å) | doppelte Glanzwinkel (2 Θ) | relative Intensität |
|---|---|---|
| 11,7826 | 7,50 | 22 |
| 5,8252 | 15,20 | 19 |
| 3,6236 | 24,55 | 18 |
| 3,4612 | 25,72 | 41 |
| 3,3081 | 26,93 | 8 |
| 3,1570 | 28,25 | 100 |
| 2,8750 | 31,08 | 32 |
| 2,7828 | 32,08 | 17 |

gekennzeichnet.

Die vorliegende Erfindung betrifft das Diketopyrrolopyrrol der Formel 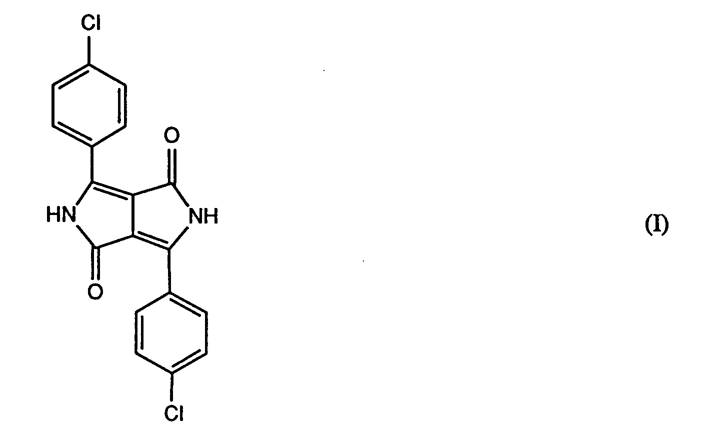 in seiner β-Modifikation, deren Röntgenbeugungsdiagramm durch folgende Beugungslinien

| Netzebenenabstände (d-Werte in Å) | doppelte Glanzwinkel (2 Θ) | relative Intensität |
|---|---|---|
| 15,2265 | 5,80 | 25 |
| 7,5110 | 11,77 | 27 |
| 6,5395 | 13,53 | 20 |
| 5,9710 | 14,82 | 42 |
| 5,0037 | 17,71 | 11 |
| 4,8711 | 18,20 | 12 |
| 3,8033 | 23,37 | 25 |
| 3,6411 | 24,43 | 14 |
| 3,2721 | 27,23 | 100 |
| 3,0229 | 29,53 | 27 |

gekennzeichnet ist.

Die Herstellung dieser neuen β-Modifikation erfolgt dadurch, dass ein lösliches Diketopyrrolopyrrol der Formel 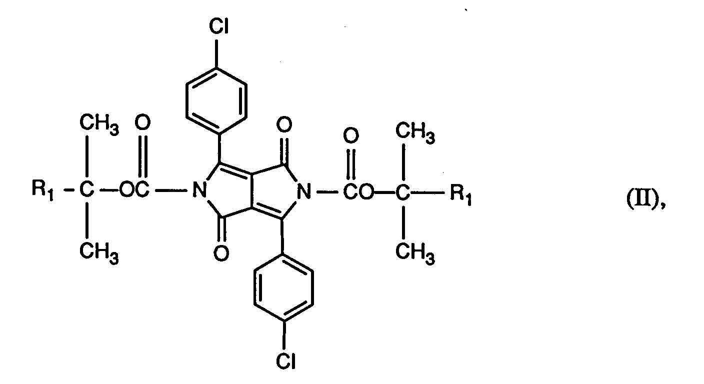 worin R₁ C₁-C₆-Alkyl ist, in Wasser und/oder einem organischen Lösungsmittel gelöst, in Gegenwart einer Säure auf einer Temperatur zwischen 50 und 150°C erhitzt wird und danach das nach dem Abkühlen ausgefallene Produkt nach üblichen Methoden isoliert wird.

Als C₁-C₆-Alkyl bedeutet R₁ z.B. Methyl, Ethyl, n-Propyl, Isopropyl, n-Butyl, tert.-Butyl, n-Amyl oder Hexyl.

Vorzugsweise bedeutet R₁ Ethyl, insbesondere aber Methyl.

Zweckmässig wird das gelöste Diketopyrrolopyrrol der Formel II in Gegenwart der Säure unter Rückfluss während 10 Minuten bis 20 Stunden, je nach Lösungsmittel, behandelt und das Gemisch danach auf 10 bis 30°C abgekühlt.

Als Lösungsmittel können Wasser oder inerte aprotische organische Lösungsmittel, wie z.B. Dimethylformamid, Tetrahydrofuran, Ethylenglykol, Ethylenglykolmonomethylether, Dodecan, Toluol, Xylol, Acetylaceton, Dimethylsulfoxid oder deren Mischungen verwendet werden. Bevorzugt werden Tetrahydrofuran oder ein Dimethylformamid/Wasser-Gemisch, insbesondere im Verhältnis von ca. 2:1.

Als Säuren eignen sich sowohl anorganische, als auch organische Säuren, wie z.B. Salzsäure, Schwefelsäure, Toluolsulfonsäure oder Trifluoressigsäure. Toluol-4-sulfonsäure ist bevorzugt. Zweckmässig werden 10 bis 30, bevorzugt 15 bis 25 Mol Säure auf 1 Mol Diketopyrrolopyrrol der Formel II eingesetzt. Die Säure kann entweder vor, zusammen mit oder nach der Pigmentsalzsuspension zugegeben werden, vorzugsweise vor oder zusammen mit der Pigmentsalzsuspension.

Bevorzugt verwendet man 15 bis 20 Mol Toluol-4-sulfonsäure, bezogen auf das Diketopyrrolopyrrol unter Rückfluss in Dimethylformamid/Wasser 2:1 während 15 bis 45 Minuten
oder insbesondere
in Tetrahydrofuran während 8 bis 16 Stunden.

Diketopyrrolopyrrole der Formel II können in Analogie zu allgemein bekannten Methoden z.B. durch Umsetzung eines Diketopyrrolopyrrols der Formel I mit einem Dicarbonat der Formel 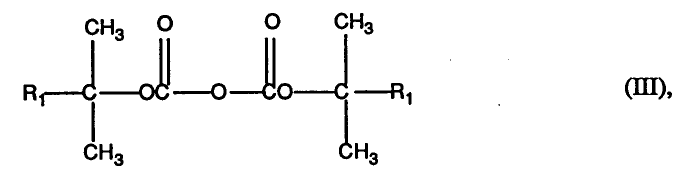 oder mit einem Trihaloessigsäureester der Formel 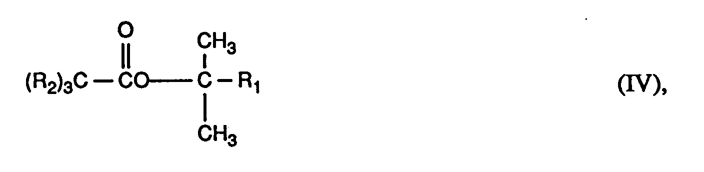 worin R₂ Chlor, Fluor oder Brom bedeutet,
oder mit einem Azid der Formel 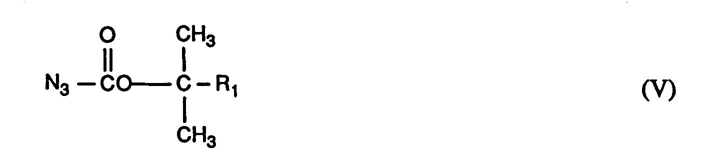 wobei R₁ jeweils die oben angegebene Bedeutung hat,
in einem aprotischen organischen Lösungsmittel in Gegenwart einer Base als Katalysator, bei Temperaturen zwischen 0 und 400°C während 2 bis 80 Stunden, erhalten werden.

Das Dicarbonat der Formel III, der Trihaloessigsäureester der Formel IV oder das Azid der Formel V wird zweckmässig in einem 2- bis 10-fachem Überschuss eingesetzt.

Bevorzugt wird das Diketopyrrolopyrrol der Formel I mit einem Dicarbonat der Formel m umgesetzt.

Dicarbonate der Formel III, Trihaloessigsäureester der Formel IV und Azide der Formel V sind bekannte Substanzen. Sollten einige noch neu sein, so können sie in Analogie zu allgemein bekannten Methoden hergestellt werden.

Als Lösungsmittel eignen sich beispielsweise Ether, wie Tetrahydrofuran oder Dioxan, oder Glykoläther, wie Ethylenglykol-methyläther, Ethylenglykol-ethylether, Diethylenglykol-monomethylether oder Diethylenglykol-monoethylether, ferner dipolar-aprotische Lösungsmittel, wie Acetonitril, Benzonitril, N,N-Dimethylformamid, N,N-Dimethylacetamid, Nitrobenzol, N-Methylpyrrolidon, halogenierte aliphatische oder aromatische Kohlenwasserstoffe, wie Trichlorethan, Benzol oder durch Alkyl, Alkoxy oder Halogen substituiertes Benzol, wie Toluol, Xylol, Anisol oder Chlorbenzol oder aromatische N-Heterocyclen, wie Pyridin, Picolin oder Chinolin. Bevorzugte Lösungsmittel sind z.B. Tetrahydrofuran, N,N-Dimethylformamid, N-Methylpyrrolidon. Die genannten Lösungsmittel können auch als Mischungen eingesetzt werden. Zweckmässigerweise verwendet man 5-20 Gew.-Teile Lösungsmittel auf 1 Gew.-Teil der Reaktionsteilnehmer.

Als Katalysator geeignete Basen sind beispielsweise die Alkalimetalle selbst, wie Lithium, Natrium oder Kalium sowie deren Hydroxyde oder Carbonate, oder Alkaliamide, wie Lithium-, Natrium- oder Kaliumamid oder Alkalihydride, wie Lithium-, Natrium-oder Kaliumhydrid, oder Erdalkali- oder Alkalialkoholate, die sich insbesondere von primären, sekundären oder tertiären aliphatischen Alkoholen mit 1 bis 10 C-Atomen ableiten, wie z.B. Lithium-, Natrium- oder Kaliummethylat, -ethylat, -n-propylat, -isopropylat, n-butylat, -sek.-butylat, tert.-butylat, -2-methyl-2-butylat, -2-methyl-2-pentylat, -3-methyl3-pentylat, -3ethyl-3-pentylat, und ferner organische aliphatische, aromatische oder heterocyclische N-Basen, darunter z.B. Diazabicyclooctan, Diazabicycloundecen und 4-Dimethylaminopyridin und Trialkylamine, wie z.B. Trimethyl- oder Triethylamin. Man kann aber auch ein Gemisch der genannten Basen verwenden.

Bevorzugt werden die organischen N-Basen, wie z.B. Diazabicyclooctan, Diazabicycloundecen und insbesondere 4-Dimethylaminopyridin.

Die Umsetzung wird bevorzugt bei Temperaturen zwischen 10 und 100°C, insbesondere zwischen 14 und 40°C, durchgeführt und zwar bei atmosphärischem Druck.

Mittlerweile ist aber auch gefunden worden, dass bei der Herstellung des Diketopyrrolopyrrols der Formel I nach dem herkömmlichen, wie z.B. im US-Patent 4 579 949 beschriebenen Verfahren, ebenfalls die β-Modifikation erhalten wird, wenn bei der Protonierung des Pigmentalkalimetallsalzes und der nachfolgenden Konditionierung ganz bestimmte Bedingungen eingehalten werden, d.h. wenn die Pigmentalkalisalz-Suspension in einem 1-2:1-Volumen-Gemisch von einem Alkohol ROH, worin R C₁-C₄-Alkyl ist, und Wasser, bei einer Temperatur zwischen -20 und 20°C, bevorzugt um 0°C, ausgetragen wird und in Gegenwart einer Säure in genügender Menge, um den pH <7 zu halten, bei der gleichen Temperatur, zweckmässig z.B. 10 Minuten bis 10 Stunden, gerührt wird.

R ist als C₁-C₄-Alkyl bevorzugt Methyl.

Geeignete Säuren sind z.B. Salzsäure, Schwefelsäure oder Essigsäure. Bevorzugt wird Schwefelsäure.

Das Röntgenbeugungsdiagramm des so erhaltenen Produkts entspricht, abgesehen von kleinen auf die beschränkte Auflösung zurückzuführenden Schwankungen in den Lagen der Reflexe, dem Röntgenbeugungsdiagramm der β-Modifikation.

Auch das erfingungsgemässe β-Diketopyrrolopyrrol eignet sich, wie bereits z.B. in den US-Patenten 4 415 685 und 4 579 949 für dessen α-Modifikation beschrieben, als Pigment zum Färben von hochmolekularem organischem Material. Da es aber beim Erhitzen auf je nach Substrat verschieden hohe Temperaturen wieder in die α-Modifikation umgewandelt wird, ist bei einer Verwendung in Materialien, die bei höheren Temperaturen verarbeitet werden, Vorsicht geboten.

Wie viele andere Pigmente, kann das erfindungsgemässe β-Diketopyrrolopyrrol auch mit Vorteil nach bekannten Methoden oberflächenbehandelt werden, um seine Eigenschaften in Lacksystemen zu verbessern. Additive, die zur Verminderung oder Vermeidung von Flokkulation, sowie zur Verbesserung der Dispersionsstabilität dienen, können vorteilhaft mit dem erfindungsgemässen Pigment verwendet werden. Das so behandelte Pigment zeigt gute Eigenschaften allein oder in Mischung mit anderen Pigmenten zur Erzeugung von roten Volltonausfärbungen in verschiedenen Lacksystemen, jedoch bevorzugt in Automobillacksystemen des Acryl-, Alkyd- und Polyestertyps. 2-Phthalimidomethylchinacridon, Chinacridonsulfonsäure und andere ähnliche Derivate können z.B. als Antiflokkulationsmittel dienen. In bestimmten Systemen kann der Zusatz von polymeren Dispergiermittel die Eigenschaften des Pigments noch zusätzlich verbessern.

Das erfindungsgemässe β-Diketopyrrolopyrrol wird in Mengen von 0,01 bis 30 Gew.-%, bevorzugt 0,1 bis 10 Gew.-% bezogen auf das zu färbende hochmolekulare organische Material eingesetzt und in diesem zweckmässig bei Temperaturen zwischen 20 und 180°C eingearbeitet.

Das erfindungsgemässe β-Diketopyrrolopyrrol lässt sich beispielsweise als Pulver, Teig, Flushpaste und Zubereitung anwenden und eignet sich z.B. für Druckfarben, Leimfarben, Binderfarben oder Lacke aller Art, wie physikalisch und oxydativ trocknende Lacke, säure-, amin- und peroxid-härtende Lacke oder Polyurethanlacke. Das Pigment kann, wenn es die Verarbeitungstemperatur zulässt, auch zum Färben von synthetischen, halbsynthetischen oder natürlichen makromolekularen Stoffen verwendet werden, eventuell zusammen mit anderen organischen oder anorgansichen Pigmenten. Die erhaltenen Färbungen, beispielsweise in Lacken, Drucken oder Kunststoffen, zeichnen sich durch einen gelbroten Farbton, gute Überlackier-, Migrations-, Licht-und Wetterechtheit sowie durch hohe Farbstärke und Transparenz aus.

Das erfindungsgemässe Pigment kann zum Färben von festen, elastischen, pastenartigen, dickflüssigen, dünnflüssigen oder thixotropen Massen verwendet und in diese nach an sich bekannten Verfahren eingearbeitet werden. Wasserhaltige Teige können beispielsweise durch Einrühren des Pigments in Wasser, gegebenenfalls unter Zusatz eines Netz-oder Dispergiermittels oder durch Einrühren oder Einkneten des Pigments in ein Dispergiermittel in Gegenwart von Wasser und gegebenenfalls von organischen Lösungsmitteln oder Ölen erhalten werden. Diese Teige können beispielsweise wiederum zur Herstellung von Flushpasten, Druckfarben, Leimfarben und Kunststoffdispersionen verwendet werden. Das Pigment kann aber auch durch Einrühren, Einwalzen, Einkneten oder Einmahlen in Wasser, organische Lösungsmittel, nicht trocknende Öle, trocknende Öle, Lacke, Kunststoffe oder Gummi gebracht werden. Schliesslich ist es auch möglich, das Pigment durch trockenes Mischen mit organischen oder anorganischen Massen, Granulaten, Faserstoffen, Pulvern und anderen Pigmenten zu Stoffmischungen zu verarbeiten.

Die nachfolgenden Beispiele erläutern die Erfindung.

### Beispiel 1a: (Herstellung des löslichen Diketopyrrolopyrrols)

Einer Suspension von 20,0 g 1,4-Diketo-3,6-di-(4-chlorphenyl)-pyrrolo[3,4-c]pyrrol in 500 ml N,N-Dimethylformamid (über Molekularsieb getrocknet) werden 1,78 g 4-Dimethylaminopyridin und danach 26,8 g Di-tert.-butyldicarbonat zugebeben. Das Reaktionsgemisch wird bei Raumtemperatur unter Ausschluss von atmosphärischer Feuchtigkeit gerührt. Nach 15 Stunden werden erneut 26,8 g Di-tert.-butyldicarbonat zugegeben und 30 Stunden weitergerührt. Das ausgefallene braun-orange Produkt wird abfiltriert, mit Methanol gewaschen und im Vakuum bei Raumtemperatur getrocknet. Man erhält 21,8 g (70 % d.Th.) des Produktes der Formel

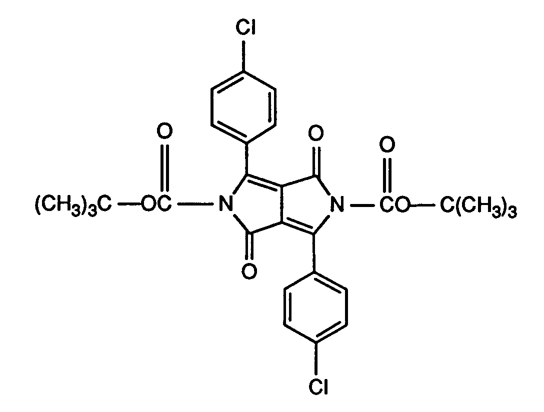

| Analyse: | C | H | N | Cl |
|---|---|---|---|---|
| Ber.: | 60,33 % | 4,70 % | 5,03 % | 12,72 % |
| Gef.: | 60,24 % | 4,79 % | 4,92 % | 12,50 % |

b) Eine Mischung aus 1,5 g des Produktes aus a) und 5,1 g Toluol-4-sulfonsäure-Monohydrat in 75 ml Tetrahydrofuran wird 15 Stunden unter Rückfluss gerührt und anschliessend auf 30°C abgekühlt. Das ausgefallene Pigment wird abfiltriert, mit Methanol und dann mit Wasser gewaschen und getrocknet. Man erhält 0,55 g (57,2 % d.Th.) eines roten Pulvers.

| Analyse: | C | H | N | Cl |
|---|---|---|---|---|
| Ber.: | 60,53 % | 2,82 % | 7,84 % | 19,85 % |
| Gef.: | 60,38 % | 2,96 % | 7,69 % | 19,42 % |

Das Röntgenbeugungsdiagramm ist durch folgende Beugungslinien

| Netzebenenabstände (d-Werte in Å) | doppelte Glanzwinkel (2 Θ) | relative Intensität |
|---|---|---|
| 15,2265 | 5,80 | 25 |
| 7,5110 | 11,77 | 27 |
| 6,5395 | 13,53 | 20 |
| 5,9710 | 14,82 | 42 |
| 5,0037 | 17,71 | 11 |
| 4,8711 | 18,20 | 12 |
| 3,8033 | 23,37 | 25 |
| 3,6411 | 24,43 | 14 |
| 3,2721 | 27,23 | 100 |
| 3,0229 | 29,53 | 27 |

gekennzeichnet.

### Beispiel 2:

In einem Sulfierkolben werden unter Stickstoff 170 ml tert.-Amylalkohol vorgelegt. Nach der Zugabe von 14,72 g Natrium wird das Gemisch auf 92-102°C aufgeheizt. Das geschmolzene Natrium wird unter heftigem Rühren über Nacht bei 100-107°C gehalten. Zur entstandenen Lösung werden dann 44,02 g 4-Chlorbenzonitril und 37,2 g Bernsteinsäurediisopropylester gelöst in 50 ml tert.-Amylalkohol bei 80°C innerhalb von 2 Stunden bei 105°C zudosiert. Das Reaktionsgemisch wird bei 101-105°C 3 Stunden weitergerührt und gleichzeitig noch 4,88 g Bernsteinsäurediisopropylester zugegeben. Anschliessend wird das Reaktionsgemisch auf Raumtemperatur abgekühlt und in eine Mischung von 270 ml Methanol, 200 ml Wasser und 64,1 g konz. Schwefelsäure bei 0°C ausgetragen und dann bei 0°C 6 Stunden gerührt. Das rote Gemisch wird abfiltriert, mit Methanol und danach mit Wasser gewaschen und bei 80°C im Vakuumtrockenschrank getrocknet. Man erhält 52,3 g eines roten Pulvers.

| Analyse: | C | H | N | Cl |
|---|---|---|---|---|
| Ber.: | 60,53 % | 2,82 % | 7,84 % | 19,85 % |
| Gef.: | 60,29 % | 2,79 % | 7,60 % | 19,24 % |

Das Röntgenbeugungsdiagramm ist durch folgende Beugungslinien

| Netzebenenabstände (d-Werte in Å) | doppelte Glanzwinkel (2 Θ) | relative Intensität |
|---|---|---|
| 15,2765 | 5,78 | 25 |
| 7,5209 | 11,76 | 27 |
| 6,5400 | 13,53 | 20 |
| 5,9813 | 14,80 | 43 |
| 5,0006 | 17,72 | 12 |
| 4,8742 | 18,19 | 12 |
| 3,8011 | 23,38 | 25 |
| 3,6402 | 24,43 | 15 |
| 3,2716 | 27,23 | 100 |
| 3,0217 | 29,53 | 27 |

gekennzeichnet.

### Beispiel 3:

1,0 g des gemäss Beispiel lb hergestellten Pigments werden mit 63,0 g Polyvinylchlorid, 3,0 g epoxydiertem Soyabohnenöl, 2,0 g Barium/Cadmium-Hitzestabilisator und 32,0 g Dioctylphthalat vermischt und auf einem Walzenstuhl während 8 Minuten bei 160°C zu einer dünnen Folie verarbeitet. Die so erzeugte rote PVC-Folie zeichnet sich durch sehr gute coloristische Eigenschaften und hervorragende Hitze-, Licht-und Migrationsechtheit aus.

### Beispiel 4:

Eine Mischung von 460 g Steatitkugeln von 8 mm Durchmesser, ein Alkydlack bestehend aus 58,7 g Alkydharz ® ALKYDAL F 310 (Bayer AG) 60 %ig in Xylol, 58,7 g Alkydharz ® ALKYDAL F 32 (Bayer AG) 60 %ig in Xylol, 2,0 g ®Silikonöl A (Bayer AG) 1 %ig in Xylol, 4,0 g n-Butanol, 4,0 g ®DOWANOL (Dow Chem.), 15 g Xylol, 5,6 g Dispergiermittel ®DISPERBYK D-160 (BYK-Chemie) und ferner 28,0 g des gemäss Beispiel 1b hergestellten Pigmentes werden in einer Glasflasche mit "Twist-off"-Verschluss während 72 Stunden auf dem Rollgestell dispergiert. Nach Zugabe von 24,0 g der Melaminkomponente ®CYMEL 327 (Cyanamid) 90 %ig in Xylol wird eine Stunde auf dem Rollgestell weiter dispergiert. Anschliessend werden die Steatitkugeln abgetrennt. Die so erhaltene Farblackanreibung wird auf ®MILAR-Transparenzfolien appliziert und anschliessend während 30 Minuten bei 130°C eingebrannt (Lackschichtdicke 50 µm). Man erhält eine rote Ausfärbung mit ausgezeichneten koloristischen Eigenschaften.

## Patentansprüche

1. Diketopyrrolopyrrol der Formel 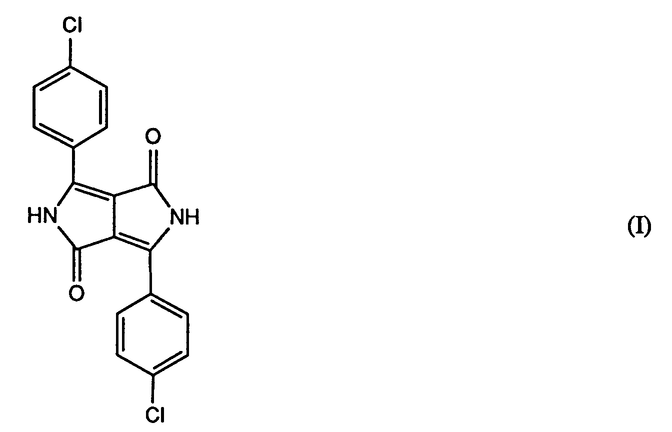 in seiner β-Modifikation, deren Röntgenbeugungsdiagramm durch folgende Beugungslinien
| Netzebenenabstände (d-Werte in Å) | doppelte Glanzwinkel (2 Θ) | relative Intensität |
|---|---|---|
| 15,2265 | 5,80 | 25 |
| 7,5110 | 11,77 | 27 |
| 6,5395 | 13,53 | 20 |
| 5,9710 | 14,82 | 42 |
| 5,0037 | 17,71 | 11 |
| 4,8711 | 18,20 | 12 |
| 3,8033 | 23,37 | 25 |
| 3,6411 | 24,43 | 14 |
| 3,2721 | 27,23 | 100 |
| 3,0229 | 29,53 | 27 |
gekennzeichnet ist.

2. Verfahren zur Herstellung des Diketopyrrolopyrrols gemäss Anspruch 1 dadurch gekennzeichnet, dass ein lösliches Diketopyrrolopyrrol der Formel 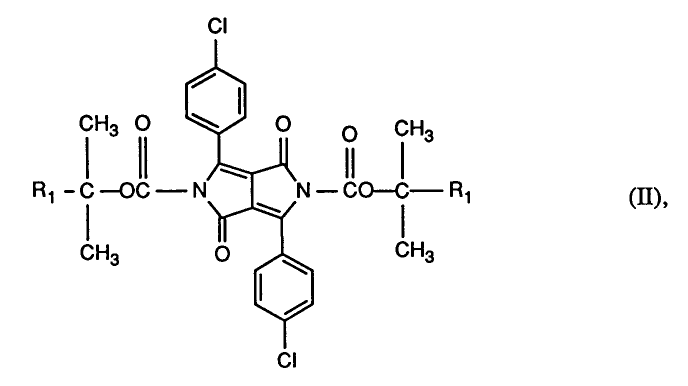 worin R₁ C₁-C₆-Alkyl ist, in Wasser oder einem organischen Lösungsmittel mit oder ohne Wasser gelöst, in Gegenwart einer Säure auf einer Temperatur zwischen 50 und 150°C erhitzt wird und danach das nach dem Abkühlen ausgefallene Produkt nach üblichen Methoden isoliert wird.

3. Verfahren gemäss Anspruch 2, dadurch gekennzeichnet, dass eine Verbindung der Formel II verwendet wird, worin R₁ Ethyl oder bevorzugt Methyl ist.

4. Verfahren gemäss Anspruch 2, dadurch gekennzeichnet, dass das Diketopyrrolopyrrol der Formel II unter Rückfluss während 10 Minuten bis 20 Stunden behandelt und das Gemisch danach auf 10 bis 30°C abgekühlt wird.

5. Verfahren gemäss Anspruch 4, dadurch gekennzeichnet, dass als Lösungsmittel Tetrahydrofuran oder ein Dimethylformamid/Wasser-Gemisch verwendet wird.

6. Verfahren gemäss Anspruch 2, dadurch gekennzeichnet, dass als Säure Toluol-4-sulfonsäure verwendet wird.

7. Verfahren gemäss Anspruch 2, dadurch gekennzeichnet, dass 10 bis 30 Mol Säure auf 1 Mol Diketopyrrolopyrrol der Formel II eingesetzt werden.

8. Verfahren gemäss Anspruch 2, dadurch gekennzeichnet, dass das Diketopyrrolopyrrol der Formel II mit 15 bis 20 Mol Toluol-4-sulfonsäure, bezogen auf das Diketopyrrolopyrrol 15 bis 45 Minuten unter Rückfluss in Dimethylformamid/Wasser 2:1 behandelt wird.

9. Verfahren gemäss Anspruch 2, dadurch gekennzeichnet, dass das Diketopyrrolopyrrol der Formel II mit 15 bis 20 Mol Toluol-4-sulfonsäure, bezogen auf das Diketopyrrolopyrrol 8 bis 16 Stunden in Tetrahydrofuran behandelt wird.

10. Hochmolekulares organisches Material enthaltend ein Diketopyrrolopyrrol gemäss Anspruch 1.

## Claims

1. A diketopyrrolopyrrole of the formula 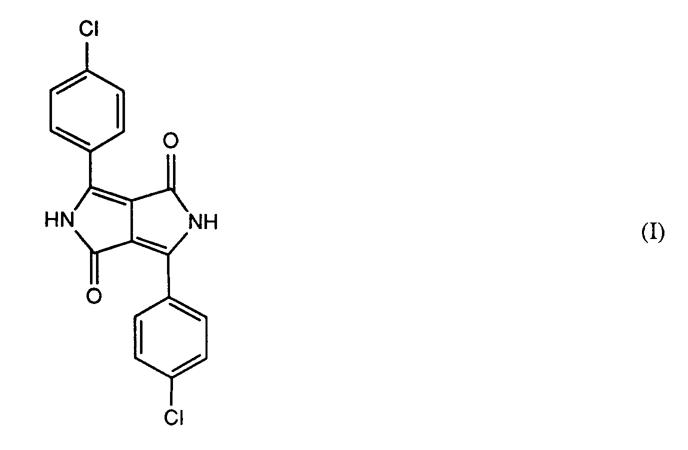 in its β-modification, whose X-ray diffraction diagram is characterized by the following diffraction lines
| interplanar spacings (d values in Å) | twice the Bragg angle (2 Θ) | relative intensity |
|---|---|---|
| 15.2265 | 5.80 | 25 |
| 7.5110 | 11.77 | 27 |
| 6.5395 | 13.53 | 20 |
| 5.9710 | 14.82 | 42 |
| 5.0037 | 17.71 | 11 |
| 4.8711 | 18.20 | 12 |
| 3.8033 | 23.37 | 25 |
| 3.6411 | 24.43 | 14 |
| 3.2721 | 27.23 | 100 |
| 3.0229 | 29.53 | 27. |

2. A process for the preparation of a diketopyrrolopyrrole according to claim 1 which comprises dissolving a soluble diketopyrrolopyrrole of the formula 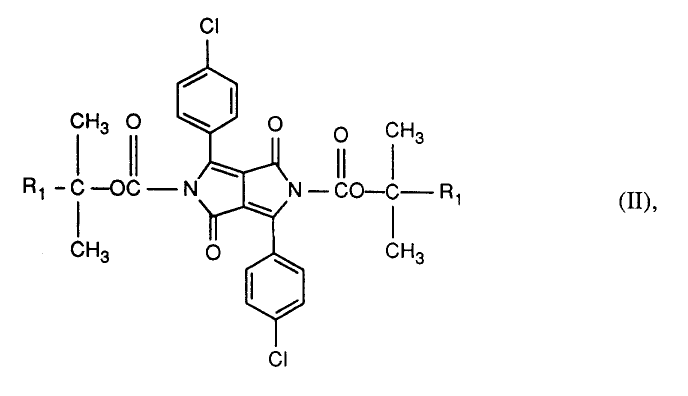 in which R₁ is C₁-C₆alkyl, in water or an organic solvent with or without water, heating the solution at a temperature of between 50 and 150°C in the presence of an acid, and then isolating the product, which has precipitated after cooling, by conventional methods.

3. A process according to claim 2, wherein a compound of the formula II is used in which R₁ is ethyl or, preferably, methyl.

4. A process according to claim 2, wherein the diketopyrrolopyrrole of the formula II is treated under reflux for from 10 minutes to 20 hours and the mixture is then cooled to from 10 to 30°C.

5. A process according to claim 4, wherein the solvent used is tetrahydrofuran or a dimethylformamide/water mixture.

6. A process according to claim 2, wherein the acid used is 4-toluenesulfonic acid.

7. A process according to claim 2, wherein from 10 to 30 mol of acid are employed per mole of diketopyrrolopyrrole of the formula II.

8. A process according to claim 2, wherein the diketopyrrolopyrrole of the formula II is treated with from 15 to 20 mol of 4-toluenesulfonic acid, based on the diketopyrrolopyrrole, under reflux in dimethylformamide/water 2:1 for from 15 to 45 minutes.

9. A process according to claim 2, wherein the diketopyrrolopyrrole of the formula II is treated with from 15 to 20 mol of 4-toluenesulfonic acid, based on the diketopyrrolopyrrole, in tetrahydrofuran for from 8 to 16 hours.

10. A high molecular weight organic material comprising a diketopyrrolopyrrole according to claim 1.

## Revendications

1. Dicétopyrrolopyrrole de formule 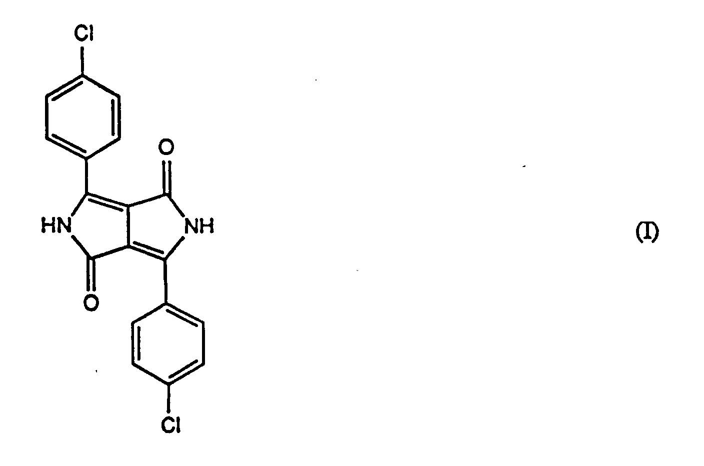 dans sa modification β, dont le diagramme de diffraction des rayons X est caractérisé par les lignes de diffraction suivantes
| Distance entre les plans réticulaires (valeurs de d en A) | Double de l'angle de Bragg (2 Θ) | Intensité relative |
|---|---|---|
| 15,2265 | 5,80 | 25 |
| 7,5110 | 11,77 | 27 |
| 6,5395 | 13,53 | 20 |
| 5,9710 | 14,82 | 42 |
| 5,0037 | 17,71 | 11 |
| 4,8711 | 18,20 | 12 |
| 3,8033 | 23,37 | 25 |
| 3,6411 | 24,43 | 14 |
| 3,2721 | 27,23 | 100 |
| 3,0229 | 29,53 | 27 |

2. Procédé de préparation du dicétopyrrolopyrrole selon la revendication 1, caractérisé en ce qu'on dissout un dicétopyrrolopyrrole soluble de formule 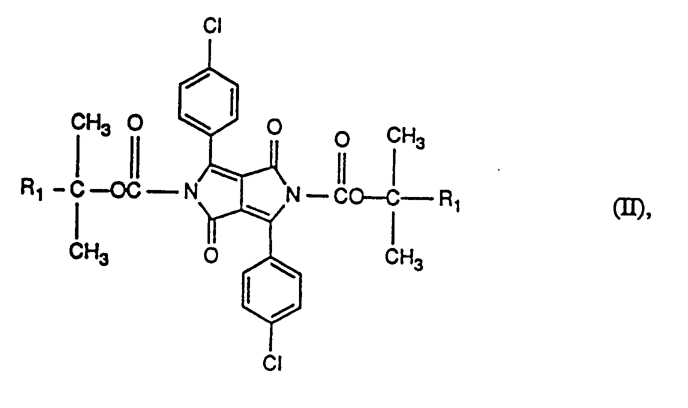 dans laquelle R₁ est un groupe alkyle en C₁-C₆, dans l'eau ou dans un solvant organique avec ou sans eau, en ce qu'on le chauffe en présence d'un acide à une température entre 50 et 150°C et on isole ensuite le produit précipité après le refroidissement selon des procédés usuels.

3. Procédé selon la revendication 2, caractérisé en ce qu'on utilise un composé de formule II dans lequel R₁ est un groupe éthyle ou de préférence méthyle.

4. Procédé selon la revendication 2, caractérisé en ce qu'on traite le dicétopyrrolopyrrole de formule II à reflux pendant 10 minutes à 20 heures et on refroidit ensuite le mélange à 10 à 30°C.

5. Procédé selon la revendication 4, caractérisé en ce qu'on utilise comme solvant le tétrahydrofurane ou un mélange diméthylformamide/eau.

6. Procédé selon la revendication 2, caractérisé en ce qu'on utilise comme acide l'acide toluène-4-sulfonique.

7. Procédé selon la revendication 2, caractérisé en ce qu'on utilise 10 à 30 moles d'acide pour 1 mole de dicétopyrrolopyrrole de formule II.

8. Procédé selon la revendication 2, caractérisé en ce qu'on traite le dicétopyrrolopyrrole de formule II avec 15 à 20 moles d'acide toluène-4-sulfonique, par rapport au dicétopyrrolopyrrole, 15 à 45 minutes à reflux dans un mélange 2:1 diméthylformamide/eau.

9. Procédé selon la revendication 2, caractérisé en ce qu'on traite le dicétopyrrolopyrrole de formule II avec 15 à 20 moles d'acide toluène-4-sulfonique, par rapport au dicétopyrrolopyrrole, 8 à 16 heures dans le tétrahydrofurane.

10. Matériau organique de poids moléculaire élevé contenant un dicétopyrrolopyrrole selon la revendication 1.
